# EUROPEAN PATENT APPLICATION

(11) **EP 4 818 167 A1**
(43) Date of publication of application: **07.10.2026**
(21) Application number: 25168526.9
(22) Date of filing: 04.04.2025
(51) Int. Cl.: C07D 413/12, A01N 43/80

(54) **PROCESS FOR THE PREPARATION OF PYROXASULFONE WITH SURFACTANTS**

(71) Applicant: Adama Agan Ltd., 7710201 Ashdod (IL)
(72) Inventor: ASHUSH, Natali, 4284200 Bat Hefer (IL); COHEN, Yair, 7685925 Mazkeret Batya (IL)
(74) Representative: Modiano, Gabriella Diana

(57) **Abstract**

The present application relates to a process for the preparation of pyroxasulfone comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst, a surfactant selected from the group of non-ionic surfactants, anionic surfactants or mixtures thereof, and water.

## Description

### FIELD OF THE INVENTION

The present invention relates to the field of synthesis of organic compounds, more specifically to a process for the preparation of pyroxasulfone.

### BACKGROUND PRIOR ART

US2023012374 discloses the preparation of pyroxasulfone (3-[[5-(difluoromethoxy)-1-methyl-3-(trifluoromethyl)pyrazol-4-yl]methylsulfonyl]-5,5-dimethyl-4H-1,2-oxazole) by oxidizing 3-[(5-difluoromethoxy-1-methyl-3-trifluoromethylpyrazol-4-yl)methylthio]-4,5-dihydro-5,5-dimethylisoxazole in the presence of a metal catalyst and hydrogen peroxide. The yields of the reaction are typically lowered by incomplete oxidation of the thio group to the corresponding sulfoxide. Different organic solvents are tested in US2023012374, for example, butyl acetate, isopropyl acetate, ethyl acetate, methanol, butanol, ethanol, acetonitrile, DMF, or NMP, each solvent and solvent amount having a different effect on the amount pyroxasulfone formed and the resulting amounts of the sulfoxide intermediate impurity. According to the tested organic solvents in US2023012374, the amount of organic solvent ranges from 0.4 to 1.8 liters of organic solvent per mole of 3-[(5-difluoromethoxy-1-methyl-3-trifluoromethylpyrazol-4-yl)methylthio]-4,5-dihydro-5,5-dimethylisoxazole.

### SUMMARY OF THE INVENTION

The present invention provides an alternative to the processes for the preparation of pyroxasulfone, wherein no organic solvent or a negligible amount of organic solvent is present during the oxidation reaction for the preparation of pyroxasulfone. According to an embodiment of this invention, conducting the oxidation in the presence of a metal catalyst and a non-ionic or anionic surfactant avoids or significantly reduces the use of organic solvents during the oxidation reaction, as comparison with the prior art, while the pyroxasulfone yield is excellent, and the amount of sulfoxide impurity is acceptable. The reduction in the amount of organic solvent means a reduction in the cost of the process, as well as the reaction waste.

Thus, the present application is directed to a process for the preparation of pyroxasulfone (3-[[5-(difluoromethoxy)-1-methyl-3-(trifluoromethyl)pyrazol-4-yl]methylsulfonyl]-5,5-dimethyl-4H-1,2-oxazole) comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst selected from the groups 5 to 6 of the periodic table, at least one surfactant selected from the group of non-ionic surfactants, anionic surfactants or mixtures thereof, and water.

The reaction can be summarized in the following equation:

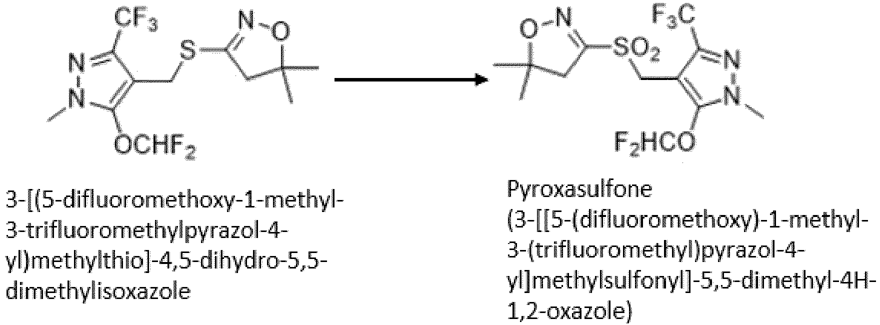

### DETAILED DESCRIPTION OF THE INVENTION

### Definitions

Embodiments of the present invention are discussed in detail below. In describing embodiments, specific terminology is employed for the sake of clarity. However, the invention is not intended to be limited to the specific terminology so selected. While specific exemplary embodiments are discussed, it should be understood that this is done for illustration purposes only. While a number of embodiments and features are described herein, it is to be understood that the various features of the invention and aspects of embodiments, even if described separately, may be combined unless mutually exclusive or contrary to the specific description. All references cited herein are incorporated by reference as if each had been individually incorporated.

As used herein, the transitional term "comprising" or "that comprises", which is synonymous with "including," or "containing," is inclusive or open-ended and does not exclude additional, un-recited elements or method steps. However, in each recitation of "comprising" herein, it is intended that the term also encompass, as alternative embodiments, the phrases "consisting essentially of" and "consisting of", where "consisting of" excludes any element or step not specified and "consisting essentially of" permits the inclusion of additional un-recited elements or steps that do not materially affect the essential or basic and novel characteristics of the composition or method under consideration.

Prior to setting forth the present subject matter in detail, it may be helpful to provide definitions of certain terms to be used herein. Unless defined otherwise, all technical and scientific terms used herein have the same meaning as is commonly understood by one of skill in the art to which this subject matter pertains.

The term "C₁-C₂₄ alkyl" means a linear or branched saturated hydrocarbon chain radical having no multiple bonds and having from one to twenty-four carbon atoms, and which is attached to the rest of the molecule by a single bond. Suitable groups include, but are not limited to, alkyl groups such as methyl, ethyl, propyl (e.g. n-propyl or iso-propyl), butyl (e.g. n-butyl, t-butyl, sec-butyl), pentyl (e.g. 1-methylpentyl, 3-methylpentyl, n-pentyl), hexyl, octyl, dodecyl, hexadecyl or octadecyl.

The term "(C₁₂-C₂₄)fatty acid methyl tauride" means a N-methyl taurate that is N-substituted with an acyl group with a linear saturated or unsaturated hydrocarbon chain with twelve to twenty-four carbon atoms. Suitable linear saturated or unsaturated hydrocarbon chains include, but are not limited to, oleyl, linoleyl, elaidyl, arachidonyl or linolenyl.

The term "a" or "an" as used herein includes the singular and the plural, unless specifically stated otherwise. Therefore, the terms "a," "an" or "at least one" can be used interchangeably in this application.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques. In this regard, used of the term "about" herein specifically includes ±10% from the indicated values in the range. In addition, the endpoints of all ranges directed to the same component or property herein are inclusive of the endpoints, are independently combinable, and include all intermediate points and ranges. Similarly, the ranges and amounts for each element of the technology described herein can be used together with ranges or amounts for any of the other elements.

### Process for the preparation of pyroxasulfone

In a first aspect, the present disclosure relates to a process for the preparation of pyroxasulfone comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst selected from the groups 5 to 6 of the periodic table, at least one surfactant selected from the group of non-ionic surfactants, anionic surfactants or mixtures thereof, and water.

This process provides an alternative to the existing processes for the preparation of pyroxasulfone, and particularly, it provides a process wherein no organic solvent, or a negligible amount of organic solvent, is present during the oxidation reaction, while the yield of pyroxasulfone is excellent, and the amount of sulfoxide impurity is less than 5%, less than 4%, in comparison with the processes where a significant proportion of organic solvent is required during the oxidation reaction for the preparation of pyroxasulfone.

The at least one surfactant is selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols; and an anionic surfactant that may be an alkali metal salt of a sulfonate surfactant, or mixtures thereof. The at least one surfactant may be selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols, or mixtures thereof.

The at least one surfactant may be selected from an anionic surfactant that may be an alkali metal salt of a sulfonate surfactant.

The at least one surfactant is selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols; an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof. The at least one surfactant may be selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols, or mixtures thereof. The at least one surfactant may be selected from an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates or (C₁₂-C₂₄)fatty acid methyl tauride, or mixtures thereof.

The at least one surfactant is selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether; an anionic surfactant that may be dioctylsulfosuccinate sodium salt, or mixtures thereof. The at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The at least one surfactant may be selected from an anionic surfactant that may be dioctylsulfosuccinate sodium salt.

The amount of the at least one surfactant selected from the group of non-ionic, anionic surfactants or mixtures thereof may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole.

The amount of the at least one surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols; and an anionic surfactant that may be an alkali metal salt of a sulfonate surfactant, or mixtures thereof. The amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols, or mixtures thereof. The amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from an anionic surfactant that may be an alkali metal salt of a sulfonate surfactant.

The amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols; an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof. The amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols, or mixtures thereof. The amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof.

The amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether; an anionic surfactant that may be dioctylsulfosuccinate sodium salt, or mixtures thereof. The amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The amount of the at least one surfactant may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be dioctylsulfosuccinate sodium salt.

The amount of water may range from 0.1 to 3, from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole. The amount of water in the process of the present invention includes water added as solvent in the reaction vessel and water included in the aqueous hydrogen peroxide solution.

The amount of water may range from 0.1 to 3, from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole and the amount of the at least one surfactant selected from the group of non-ionic surfactants, anionic surfactants or mixtures thereof, may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole. The amount of water may range from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole and the amount of the at least one surfactant selected from the group of non-ionic surfactants, anionic surfactants or mixtures thereof, may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole. The amount of water may range from 0.3 to 1 liters per mole of 3-[(5-difluoromethoxy-1-methyl-3-trifluoromethylpyrazol-4-yl)methylthio]-4,5-dihydro-5,5-dimethylisoxazole and the amount of the at least one surfactant selected from the group of non-ionic surfactants, anionic surfactants or mixtures thereof may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole.

The amount of water may range from 0.1 to 3, from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols; and an anionic surfactant that may be an alkali metal salt of a sulfonate surfactant, or mixtures thereof. The amount of water may range from 0.1 to 3, from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols, or mixtures thereof. The amount of water may range from 0.1 to 3, from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from an anionic surfactant that may be an alkali metal salt of a sulfonate surfactant.

The amount of water may range from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols; an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof. The amount of water may range from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols, or mixtures thereof. The amount of water may range from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof.

The amount of water may range from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether; an anionic surfactant that may be dioctylsulfosuccinate sodium salt, or mixtures thereof. The amount of water may range from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The amount of water may range from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The amount of water may range from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be dioctylsulfosuccinate sodium salt.

The total amount of organic solvents in the reaction may be less than 0.15 liters, less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole.

The total amount of organic solvents in the reaction may be less than 0.15 liters, less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, and the amount of the at least one surfactant selected from the group of non-ionic, anionic surfactants or mixtures thereof may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole. The total amount of organic solvents in the reaction may be less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, and the amount of the at least one surfactant selected from the group of non-ionic, anionic surfactants or mixtures thereof may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole. The total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, and the amount of the at least one surfactant selected from the group of non-ionic, anionic surfactants or mixtures thereof may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole.

The total amount of organic solvents in the reaction may be less than 0.15 liters, less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols; and an anionic surfactant that may be an alkali metal salt of a sulfonate surfactant, or mixtures thereof. The total amount of organic solvents in the reaction may be less than 0.15 liters, less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols, or mixtures thereof. The total amount of organic solvents in the reaction may be less than 0.15 liters, less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from an anionic surfactant that may be an alkali metal salt of a sulfonate surfactant.

The total amount of organic solvents in the reaction may be less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols; an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof. The total amount of organic solvents in the reaction may be less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols, or mixtures thereof. The total amount of organic solvents in the reaction may be less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof.

The total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether; an anionic surfactant that may be dioctylsulfosuccinate sodium salt, or mixtures thereof. The total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be dioctylsulfosuccinate sodium salt.

The total amount of organic solvents in the reaction may be less than 0.15 liters, less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of water may range from 0.1 to 3, from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols; and an anionic surfactant that may be an alkali metal salt of a sulfonate surfactant, or mixtures thereof. The total amount of organic solvents in the reaction may be less than 0.15 liters, less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of water may range from 0.1 to 3, from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols, or mixtures thereof. The total amount of organic solvents in the reaction may be less than 0.15 liters, less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of water may range from 0.1 to 3, from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from an anionic surfactant that may be an alkali metal salt of a sulfonate surfactant.

The total amount of organic solvents in the reaction may be less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of water may range from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols; an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof. The total amount of organic solvents in the reaction may be less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of water may range from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols, or mixtures thereof. The total amount of organic solvents in the reaction may be less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of water may range from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof.

The total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of water may range from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether; an anionic surfactant that may be dioctylsulfosuccinate sodium salt, or mixtures thereof. The total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of water may range from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of water may range from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of water may range from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be dioctylsulfosuccinate sodium salt.

The hydrogen peroxide in the process of the invention may be a 10 to 70 wt %, 20 to 65%, 25% to 60% aqueous hydrogen peroxide solution.

The amount of hydrogen peroxide may range from 2 to 9, from 2 to 7, from 2 to 5 moles per mole of 3-[(5-difluoromethoxy-1-methyl-3-trifluoromethylpyrazol-4-yl)methylthio]-4,5-dihydro-5,5-dimethylisoxazole.

The amount of hydrogen peroxide may range from 2 to 9, from 2 to 7, from 2 to 5 moles per mole of 3-[(5-difluoromethoxy-1-methyl-3-trifluoromethylpyrazol-4-yl)methylthio]-4,5-dihydro-5,5-dimethylisoxazole and the hydrogen peroxide may be a 10 to 70 wt %, 20 to 65%, 25% to 60% aqueous hydrogen peroxide solution. The amount of hydrogen peroxide may range from 2 to 7, from 2 to 5 moles per mole of 3-[(5-difluoromethoxy-1-methyl-3-trifluoromethylpyrazol-4-yl)methylthio]-4,5-dihydro-5,5-dimethylisoxazole and the hydrogen peroxide may be a 20 to 65%, 25% to 60% aqueous hydrogen peroxide solution. The amount of hydrogen peroxide may range from 2 to 5 moles per mole of 3-[(5-difluoromethoxy-1-methyl-3-trifluoromethylpyrazol-4-yl)methylthio]-4,5-dihydro-5,5-dimethylisoxazole and the hydrogen peroxide may be a 25% to 60% aqueous hydrogen peroxide solution.

The metal catalyst selected from the groups 5 to 6 of the periodic table may be selected from a tungsten catalyst, a molybdenum catalyst and a niobium catalyst. The tungsten catalyst may be selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or a mixture thereof. The tungsten catalyst may be selected from the group of tungstic acid, alkali or alkaline earth salt of tungstic acid, metal tungsten, tungsten oxide, tungsten carbide, or a mixture thereof. The molybdenum catalyst may be selected from the group of molybdic acid, molybdic acid salt, metal molybdenum, molybdenum oxide, molybdenum carbide, molybdenum chloride, or a mixture thereof. The niobium catalyst may be selected from the group of niobic acid, niobic acid salt, metal niobium, niobium oxide, niobium carbide, niobium chloride, or a mixture thereof. The metal catalyst selected from the groups 5 to 6 of the periodic table may be sodium tungstate dihydrate or ammonium molybdate tetrahydrate.

The amount of metal catalyst selected from the groups 5 to 6 of the periodic table may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole.

The amount of metal catalyst may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be selected from a tungsten catalyst, a molybdenum catalyst and a niobium catalyst. The amount of metal catalyst may range from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be sodium tungstate dihydrate or ammonium molybdate tetrahydrate.

The amount of metal catalyst may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be selected from a tungsten catalyst, a molybdenum catalyst and a niobium catalyst, and the amount of the at least one surfactant selected from the group of non-ionic, anionic surfactants or mixtures thereof may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole. The amount of metal catalyst may range from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, and the amount of the at least one surfactant selected from the group of non-ionic, anionic surfactants or mixtures thereof may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof, and the amount of the at least one surfactant selected from the group of non-ionic, anionic surfactants or mixtures thereof may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be sodium tungstate dihydrate or ammonium molybdate tetrahydrate, and the amount of the at least one surfactant selected from the group of non-ionic, anionic surfactants or mixtures thereof may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole.

The amount of metal catalyst may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be selected from a tungsten catalyst, a molybdenum catalyst and a niobium catalyst, the amount of the at least one surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols; and an anionic surfactant that may be an alkali metal salt of a sulfonate surfactant, or mixtures thereof. The amount of metal catalyst may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be selected from a tungsten catalyst, a molybdenum catalyst and a niobium catalyst, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols, or mixtures thereof. The amount of metal catalyst may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be selected from a tungsten catalyst, a molybdenum catalyst and a niobium catalyst, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from an anionic surfactant that may be an alkali metal salt of a sulfonate surfactant.

The amount of metal catalyst may range from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols; an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof. The amount of metal catalyst may range from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols, or mixtures thereof. The amount of metal catalyst may range from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof.

The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether; an anionic surfactant that may be dioctylsulfosuccinate sodium salt, or mixtures thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof, the amount of the at least one surfactant may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be dioctylsulfosuccinate sodium salt.

The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be sodium tungstate dihydrate or ammonium molybdate tetrahydrate, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether; an anionic surfactant that may be dioctylsulfosuccinate sodium salt, or mixtures thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be sodium tungstate dihydrate or ammonium molybdate tetrahydrate, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be sodium tungstate dihydrate or ammonium molybdate tetrahydrate, the amount of the at least one surfactant may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be sodium tungstate dihydrate or ammonium molybdate tetrahydrate, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be dioctylsulfosuccinate sodium salt.

The amount of metal catalyst may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be selected from a tungsten catalyst, a molybdenum catalyst and a niobium catalyst, the total amount of organic solvents in the reaction may be less than 0.15 liters, less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols; and an anionic surfactant that may be an alkali metal salt of a sulfonate surfactant, or mixtures thereof. The amount of metal catalyst may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be selected from a tungsten catalyst, a molybdenum catalyst and a niobium catalyst, the total amount of organic solvents in the reaction may be less than 0.15 liters, less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols, or mixtures thereof. The amount of metal catalyst may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be selected from a tungsten catalyst, a molybdenum catalyst and a niobium catalyst, the total amount of organic solvents in the reaction may be less than 0.15 liters, less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from an anionic surfactant that may be an alkali metal salt of a sulfonate surfactant.

The amount of metal catalyst may range from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the total amount of organic solvents in the reaction may be less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols; an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof. The amount of metal catalyst may range from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the total amount of organic solvents in the reaction may be less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols, or mixtures thereof. The amount of metal catalyst may range from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the total amount of organic solvents in the reaction may be less than 0.10 liters, less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof.

The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof, the total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether; an anionic surfactant that may be dioctylsulfosuccinate sodium salt, or mixtures thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof, the total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof, the total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be sodium tungstate dihydrate or ammonium molybdate tetrahydrate, the total amount of organic solvents in the reaction may be less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be dioctylsulfosuccinate sodium salt.

The temperature of the reaction in the process of the present invention may range from 30°C to 120°C, from 40°C to 110°C, from 50°C to 100°C.

The reaction in the process of the present invention may be performed in a time that ranges from 1 hour to 24 hours, from 2 hours to 16 hours, from 3 hours to 12 hours.

The reaction in the process of the present invention may be performed in a time that ranges from 1 hour to 24 hours, from 2 hours to 16 hours, from 3 hours to 12 hours and the temperature of the reaction may range from 30°C to 120°C, from 40°C to 110°C, from 50°C to 100°C. The reaction in the process of the present invention may be performed in a time that ranges from 2 hours to 16 hours, from 3 hours to 12 hours and the temperature of the reaction may range from 40°C to 110°C, from 50°C to 100°C. The reaction in the process of the present invention may be performed in a time that ranges from 3 hours to 12 hours and the temperature of the reaction may range from 50°C to 100°C.

The process of the present invention may further comprise a phase transfer catalyst. The process of the present invention may further comprise a phase transfer catalyst selected from the group of tetrabutylammonium chloride, tetrabutylammonium bromide, tetrabutylammonium hydrogen sulfate, tetraoctyl ammonium bromide, tetraoctyl ammonium bromide, methyltrialkyl ammonium chloride (Adogen 464), tetradecyltrimethyl ammonium bromide, methyltrioctyl ammonium chloride (Aliquat 336), didecyldimethyl ammonium chloride, benzyltrimethyl ammonium chloride, benzyltriethyl ammonium chloride, octyltrimethyl ammonium chloride, octyltrimethyl ammonium bromide, trioctylmethyl ammonium chloride, trioctylmethyl ammonium bromide, tetrabutyl phosphonium bromide, tetraoctyl phosphonium bromide or tetraphenyl phosphonium bromide.

The amount of the further phase transfer catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole.

The process of the present invention may further comprise an acid catalyst. The process of the present invention may further comprise an acid catalyst selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate.

The amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole.

The amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate. The amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid. The amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid.

The metal catalyst selected from the groups 5 to 6 of the periodic table may be selected from a tungsten catalyst, a molybdenum catalyst and a niobium catalyst, the amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate. The metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid. The metal catalyst selected from the groups 5 to 6 of the periodic table may be sodium tungstate dihydrate or ammonium molybdate tetrahydrate, the amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid.

The amount of metal catalyst may range from 0.001 to 0.1, from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be selected from a tungsten catalyst, a molybdenum catalyst and a niobium catalyst, the amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate. The amount of metal catalyst may range from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid. The amount of metal catalyst may range from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be sodium tungstate dihydrate or ammonium molybdate tetrahydrate, the amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid.

The amount of metal catalyst may range from 0.001 to 0.1, from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be selected from a tungsten catalyst, a molybdenum catalyst and a niobium catalyst, the amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate, the amount of the at least one surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols; and an anionic surfactant that may be an alkali metal salt of a sulfonate surfactant, or mixtures thereof. The amount of metal catalyst may range from 0.001 to 0.1, from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be selected from a tungsten catalyst, a molybdenum catalyst and a niobium catalyst, the amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols, or mixtures thereof. The amount of metal catalyst may range from 0.001 to 0.1, from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be selected from a tungsten catalyst, a molybdenum catalyst and a niobium catalyst, the amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from an anionic surfactant that may be an alkali metal salt of a sulfonate surfactant.

The amount of metal catalyst may range from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols; an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof. The amount of metal catalyst may range from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols, or mixtures thereof. The amount of metal catalyst may range from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, the amount of the at least one surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride, or mixtures thereof.

The amount of metal catalyst may range from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof, the amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant is selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether; an anionic surfactant that may be dioctylsulfosuccinate sodium salt, or mixtures thereof. The amount of metal catalyst may range from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof, the amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The amount of metal catalyst may range from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof, the amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid, the amount of the at least one surfactant may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be selected from a non-ionic surfactant that may be selected from the group consisting of polyethylene glycol (3,5-dimethyl-1-isobutylhexyl)ethers, polyethylene glycol-polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether, or mixtures thereof. The amount of metal catalyst may range from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be sodium tungstate dihydrate or ammonium molybdate tetrahydrate, the amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid, the amount of the at least one surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one surfactant may be an anionic surfactant that may be dioctylsulfosuccinate sodium salt.

The process of the present invention may comprise the following steps: i) addition of 3-[(5-difluoromethoxy-1-methyl-3-trifluoromethylpyrazol-4-yl)methylthio]-4,5-dihydro-5,5-dimethylisoxazole, addition of the at least one non-ionic or anionic surfactant or mixtures thereof, and optional addition of the metal catalyst, water and organic solvent to the reaction vessel; ii) addition of the aqueous hydrogen peroxide solution; iii) optional addition of the metal catalyst, water and organic solvent; iv) isolation and purification of the obtained pyroxasulfone from the reaction mixture, with the proviso that the metal catalyst selected from the groups 5 to 6 of the periodic table is added at least in one of the steps i) or iii).

The metal catalyst selected from the groups 5 to 6 of the periodic table may be added to the reaction vessel before and/or after the addition of the aqueous hydrogen peroxide solution. Water as solvent may be added to the reaction vessel before and/or after the addition of the aqueous hydrogen peroxide solution, or it may not be added to the reaction vessel. The organic solvent may be added to the reaction vessel before and/or after the addition of the aqueous hydrogen peroxide solution, or it may not be added to the reaction vessel.

The metal catalyst selected from the groups 5 to 6 of the periodic table may be added to the reaction vessel as solid or pre-dissolved in water or in the organic solvent, it may be added in one, two, or several portions, or it may be added in continuous way.

The aqueous hydrogen peroxide solution may be added at once, in several portions, or it may be added dropwise.

The reaction of the process of the present invention may be carried out under high-shear mixing. The under high-shear mixing may be carried out at between 2500 rpm and 30000 rpm.

Previously to the isolation and purification of the obtained pyroxasulfone from the reaction mixture, any possible unreacted hydrogen peroxide may be decomposed by treating the reaction mixture with any reducing agent known by the person skilled in the art, like for example and in a not limiting way, sodium sulfite.

The isolation, purification or removal of unreacted reactants, reagents, additives and/or impurities from pyroxasulfone may be performed by any method known by the person skilled in the art, like for example and in a not limiting way, dissolution in organic solvent, decantation, extraction, washing with aqueous or organic solvent, or crystallization in organic solvent.

A second aspect of the invention is the use of pyroxasulfone obtained according to the process hereinabove disclosed in the preparation of an agrochemical composition.

### Examples

### General procedure for the oxidation with hydrogen peroxide, sodium tungstate as catalyst, and a non-ionic or anionic surfactant.

3-[(5-difluoromethoxy-1-methyl-3-trifluoromethylpyrazol-4-yl)methylthio]-4,5-dihydro-5,5-dimethylisoxazole is added to a 1-liter reactor at 60 °C followed by addition of water and stirring is started. The surfactant is added, if needed dissolved in 10 ml of water. A solution of catalyst is prepared as follows: Sodium tungstate dihydrate is dissolved in water. Half of the catalyst solution is added to the reactor. Then, the reaction is fed with hydrogen peroxide (30%, 4.2 eq.) dropwise for 2 hrs. At the beginning of the addition a slight exotherm is observed, and after the addition of the 10% of the hydrogen peroxide, the temperature is increased to 90 to 95 °C. After the addition of hydrogen peroxide, the second half of the catalyst solution is added. After aging of additional 4 hours at 90 to 95 °C under stirring, the yields of sulfoxide impurity, 3-[(5-difluoromethoxy-1-methyl-3-trifluoromethylpyrazol-4-yl)methylsulfoxide]-4,5-dihydro-5,5-dimethylisoxazole, and pyroxasulfone are measured by the relative HPLC area. Then, the aqueous phase is removed by decantation from the reactor, and the obtained pellets of pyroxasulfone are subjected to work-up in organic solvent.

M359 is the compound 3-[(5-difluoromethoxy-1-methyl-3-trifluoromethylpyrazol-4-yl)methylthio]-4,5-dihydro-5,5-dimethylisoxazole.

M375 is the sulfoxide impurity, 3-[(5-difluoromethoxy-1-methyl-3-trifluoromethylpyrazol-4-yl)methylsulfoxide]-4,5-dihydro-5,5-dimethylisoxazole.

| Surfactant | CAS number | Purity (%) |
|---|---|---|
| Brij 35 | 9002-92-0 | 100 |
| Ecosurf EH-6 | 64366-70-7 | 100 |
| Aerosol OT-100 | 577-11-7 | 100 |
| PEG 2000 | 24991-55-7 | 100 |
| Emulsogen EL-360 | 61791-12-6 | 100 |
| Soprophor TS-54 | 104376-75-2 | 100 |
| Ecosurf EH-14 | 64366-70-7 | 90 |
| Ecosurf EH-3 | 64366-70-7 | 100 |
| Emulsogen EL-200 | 61791-12-6 | 100 |

| Experiment | Amount M359 | Surfactant | % wt of surfactant vs M359 | Surfactant dissolved in 10 ml water? | Amount Na₂WO₄·2H₂O | Amount water as solvent | Amount 30% aq. H₂O₂ solution | High-shear mixer? | % M375 after 6 hr reaction | Pyroxasulfone % after 6 hr reaction |
|---|---|---|---|---|---|---|---|---|---|---|
| 1 | 73.12 g | Brij 35 | 3.42 | No | 1.38 g | 103.62 g | 100.2 g | No | 0.85 | 99.15 |
| 2 | 73.12 g | Ecosurf EH-6 | 13.67 | No | 1.38 g | 103.62 g | 100.2 g | No | 1.04 | 98.96 |
| 3 | 73.12 g | Ecosurf EH-6 | 2.74 | No | 1.38 g | 103.62 g | 100.2 g | No | 1.6 | 98.4 |
| 4 | 73.12 g | Ecosurf EH-6 | 1.37 | No | 1.38 g | 103.62 g | 100.2 g | No | 2.01 | 97.99 |
| 5 | 73.12 g | Ecosurf EH-6 | 1.37 | No | 1.38 g | 103.62 g | 100.2 g | 4000 to 20000 rpm | 1.42 | 98.58 |
| 6 | 73.12 g | Aerosol OT-100 | 1.37 | No | 1.38 g | 103.62 g | 100.2 g | No | 2.02 | 97.98 |
| 7 | 73.12 g | PEG 2000 | 1.37 | Yes | 1.38 g | 113.62 g | 100.2 g | No | 2.4 | 97.6 |
| 8 | 375 g | Ecosurf EH-6 | 2.67 | No | 10.4 g | 520 g | 506 g | No | 1.42 | 98.58 |
| 9 | 73.12 g | PEG 2000 | 13.67 | Yes | 1.38 g | 113.62 g | 100.2 g | No | 1.4 | 98.6 |
| 10 | 73.12 g | Emulsogen EL-360 | 2.74 | Yes | 1.38 g | 113.62 g | 100.2 g | No | 1.28 | 98.72 |
| 11 | 73.12 g | Soprophor TS-54 | 2.74 | No | 1.38 g | 103.62 g | 100.2 g | No | 3.62 | 96.08 |
| 12 | 73.12 g | Ecosurf EH-14 | 2.74 | No | 1.38 g | 103.62 g | 100.2 g | No | 2.3 | 97.7 |
| 13 | 73.12 g | Emulsogen EL-360 | 2.74 | No | 1.38 g | 113.62 g | 100.2 g | No | 2.0 | 98.0 |
| 14 | 73.12 g | Ecosurf EH-3 | 2.74 | No | 1.38 g | 103.62 g | 100.2 g | No | 3.58 | 96.42 |
| 15 | 73.12 g | Emulsogen EL-200 | 2.74 | No | 1.38 g | 113.62 g | 100.2 g | No | 4.13 | 95.87 |
| 16 | 73.12 g | Alkamuls 14R | 2.74 | No | 1.38 g | 103.62 g | 100.2 g | No | 4.78 | 95.22 |
| 17 | 73.12 g | Aerosol OT-100 | 2.74 | No | 1.38 g | 103.62 g | 100.2 g | No | 1.76 | 98.24 |

## Claims

1. A process for the preparation of pyroxasulfone comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst selected from the groups 5 to 6 of the periodic table, at least one surfactant selected from the group of non-ionic surfactants, anionic surfactants or mixtures thereof, and water.

2. The process according to claim 1, wherein the non-ionic surfactant is selected from the group consisting of ethoxylated (C₆-C₁₈)-secondary alcohols, ethoxylated and propoxylated (C₈-C₁₈)-secondary alcohols, ethoxylated tristyrylphenols, ethoxylated castor oil, polyethylene glycol, ethoxylated and/or propoxylated linear or branched (C₁-C₂₄)-alkyl alcohols; and the anionic surfactant is an alkali metal salt of a sulfonate surfactant.

3. The process according to claim 2, wherein the non-ionic surfactant is selected from the group consisting of ethoxylated (C₆-C₁₄)-secondary alcohols, ethoxylated and propoxylated (C₈-C₁₆)-secondary alcohols, ethoxylated tristyrylphenols, ethoxylated castor oil, polyethylene glycol, ethoxylated and/or propoxylated linear or branched (C₄-C₂₄)alkyl alcohols; and the anionic surfactant is selected from the group of alkali metal salts of (C₁-C₂₄)alkylbenzene sulfonate, (C₁-C₂₄)alkyldiphenyloxide disulfonate, (C₁-C₂₄)dialkylsulfosuccinate, (C₁-C₂₄)alkylnaphthalene sulfonates, polynaphthalene sulfonates, lignosulfonates, (C₁₂-C₂₄)fatty acid methyl tauride.

4. The process according to claim 3, wherein the non-ionic surfactant is selected from the group consisting of polyethylene glycol (3,5-dimethyl-1-isobutylhexyl)ethers, polyethylene glycol - polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether; and the anionic surfactant is dioctylsulfosuccinate sodium salt.

5. The process according to claim 4, wherein the non-ionic surfactant is selected from the group consisting of polyethylene glycol (3,5-dimethyl-1-isobutylhexyl)ethers, polyethylene glycol - polypropylene glycol ethylhexyl ethers, ethoxylated tristyrylphenols, polyethylene glycol(10 to 60) castor oil, polyethylene glycol of molecular weight in the range from 1800 to 2200 g/mol, and polyethylene glycol(4 to 23) lauryl ether.

6. The process according to claim 4, wherein the anionic surfactant is dioctylsulfosuccinate sodium salt.

7. The process according to any of the previous claims, wherein the amount of the at least one surfactant ranges from 0.1% to 16% by weight of surfactant vs weight of 3-[(5-difluoromethoxy-1-methyl-3-trifluoromethylpyrazol-4-yl)methylthio]-4,5-dihydro-5,5-dimethylisoxazole.

8. The process according to any of the previous claims, wherein the total amount of organic solvents in the reaction is less than 0.15 liters of organic solvents per mole of 3-[(5-difluoromethoxy-1-methyl-3-trifluoromethylpyrazol-4-yl)methylthio]-4,5-dihydro-5,5-dimethylisoxazole.

9. The process according to any of the previous claims, wherein the amount of water ranges from 0.1 to 3 liters per mole of 3-[(5-difluoromethoxy-1-methyl-3-trifluoromethylpyrazol-4-yl)methylthio]-4,5-dihydro-5,5-dimethylisoxazole.

10. The process according to any of the previous claims, wherein the hydrogen peroxide is a 10 to 70 wt percent aqueous hydrogen peroxide solution.

11. The process according to any of the previous claims, wherein the metal catalyst selected from the groups 5 to 6 of the periodic table is selected from a tungsten catalyst, a molybdenum catalyst and a niobium catalyst.

12. The process according to claim 11, wherein the metal catalyst selected from the groups 5 to 6 of the periodic table is a tungsten catalyst.

13. The process according to claim 12, wherein the metal catalyst selected from the groups 5 to 6 of the periodic table is selected from the group consisting of tungstic acid, a tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or a mixture of them.

14. The process according to claim 11, wherein the metal catalyst selected from the groups 5 to 6 of the periodic table is sodium tungstate dihydrate or ammonium molybdate tetrahydrate.

15. The process according to any of the previous claims, wherein the reaction is under high-shear mixing.
